# EUROPÄISCHE PATENTSCHRIFT

(11) **EP 1 313 467 B1**
(45) Veröffentlichungstag und Bekanntmachung des Hinweises auf die Patenterteilung: **12.10.2005**
(21) Anmeldenummer: 01969617.8
(22) Anmeldetag: 21.08.2001
(51) Int. Cl.: A61K 31/381, A61K 9/00, A61K 47/14, A61P 25/14, A61P 25/16

(54) **NEUE PHARMAZEUTISCHE ZUSAMMENSETZUNG ZUR VERABREICHUNG VON N-0923**
NOVEL PHARMACEUTICAL COMPOSITION FOR ADMINISTERING N-0923
NOUVELLE COMPOSITION PHARMACEUTIQUE PERMETTANT L'ADMINISTRATION DE N-0923

(30) Priorität: 24.08.2000 DE 10041479
(43) Veröffentlichungstag der Anmeldung: 28.05.2003
(73) Patentinhaber: SCHWARZ PHARMA AG, D-40789 Monheim/Rhld. (DE)
(72) Erfinder: RIMPLER, Stephan, 40723 Hilden (DE); GRAPATIN, Sabine, 40764 Langenfeld (DE); KREIN, Cliff, 51491 Overath (DE); THELEN, Markus, 40789 Monheim (DE)
(86) Internationale Anmeldenummer: PCT/EP2001/009595
(87) Internationale Veröffentlichungsnummer: WO 2002/015903

(56) Entgegenhaltungen:
- EP-A- 0 230 629
- WO-A-01/13903
- WO-A-89/12445
- WO-A-90/13294
- WO-A-97/29735
- WO-A-99/49844
- WO-A-99/49852

## Beschreibung

### Kurzbeschreibung der Erfindung:

Die vorliegende Erfindung betrifft eine injizierbare pharmazeutische Zusammensetzung zur Verabreichung des Dopamin-Agonisten Rotigotin (N-0923) in Depotform.

Bevorzugte Ausführungsformen sind ölige Suspensionen, in denen der Wirkstoff Rotigotin in fester Phase vorliegt, sowie wasserfreie pharmazeutische Zubereitungen von Rotigotin.

Schließlich betrifft die Erfindung die Verwendung von festem Rotigotin zur Herstellung hitzesterilisierbarer Arzneimittel.

### Technischer Hintergrund:

Rotigotin (Rotigotin; S(-)-2-(N-propyl-N-2-thienylethylamino)-5-hyroxytetralin) ist ein potenter und selektiver Dopamin D2 Agonist mit therapeutischer Bedeutung in allen Erkrankungen, die mit einem gestörten Dopamin-Stoffwechsel verbunden sind, z.B. Morbus Parkinson und Restless Leg. In der Vergangenheit wurden verschiedene Versuche unternommen, Rotigotin in therapeutisch relevanten Mengen zu verabreichen.

Dabei zeigte sich jedoch, dass wegen eines ausgeprägten First-Pass-Effekts die Bioverfügbarkeit nach oraler Dosierung nur bei etwa 0.5 % liegt (Swart and Zeeuw, Pharmazie 47 (1992) 613), so daß orale Verabreichungsformen für Rotigotin nicht in Frage kommen.

Darüberhinaus unterliegt die Substanz auch bei parenteraler Verabreichung in wässriger Lösung einer raschen Eliminierung. Die Halbwertszeit von Rotigotin nach intravenöser Gabe wässriger Lösungen von Rotigotin bei Affen liegt bei 52 Minuten (Walters et al, J Pharmac Sci 83 (1994) 758), was zur Notwendigkeit einer für die Patienten inakzeptabel häufigen Verabreichung während einer Dauertherapie führen würde. Beispielsweise wird Rotigotin gemäß der WO 90/13294 durch fünfmalige intraperitoneale Gabe pro Tag oder durch Dauerinfusion verabreicht.

Auch eine subkutane Gabe einer wässrigen Lösung (5% Dextrose) von Rotigotin führte zur sehr kurzen Wirkdauer von 60-70 Minuten (Belluzzi, Movement Disorders, 9.2 (1994) 147).

Daher besteht ein Bedarf an nicht-oralen Arzneiformen für Rotigotin, durch deren Formulierung die erforderliche therapeutische Applikationsfrequenz signifikant gesenkt wird.

Aus diesem Grund wurden in jüngster Zeit transdermale Systeme entwickelt. WO 94/07468 beschreibt eine Zweiphasenmatrix, die Rotigotin als Wirkstoff enthalten kann. WO 99/49852 offenbart eine Einphasenmatrix zur transdermalen Verabreichung von Rotigotin. WO 99/49844 beschreibt ebenfalls ein transdermales System zur Verabreichung von Rotigotin.

Transdermale Systeme sind jedoch nicht für alle Patienten geeignet und weisen grundsätzlich eine Reihe inhärenter Nachteile auf. So reagieren zahlreiche Patienten auf die in Pflastern enthaltenen Inhaltsstoffe wie Kleber, Penetrationsenhancer und Polymere allergisch.

Weiterhin unterliegt die Akzeptanz von Pflastern in Abhängigkeit von nationalen Traditionen und ethnischen Besonderheiten einer großen Schwankungsbreite.

Schließlich ist es mit transdermalen Systemen nur begrenzt möglich, individuelle Dosierungen einzustellen.

Ziel der vorliegenden Erfindung war es daher, eine geeignete, aus möglichst wenig Komponenten aufgebaute alternative pharmazeutische Formulierung bereitzustellen, die die oben beschriebenen Nachteile der transdermalen und oralen Verabreichung, wie schlech te Bioverfügbarkeit, hohe Einnahmefrequenz, immunogenes Potenzial, potentielle Toxizität und schlechte individuelle Dosierbarkeit vermeidet.

Die Aufgabe wurde erfindungsgemäß gelöst durch die erstmalige Bereitstellung einer pharmazeutischen Formulierung des Wirkstoffs Rotigotin als injizierbare Depotform, die geeignet ist, Rotigotin über einen Zeitraum von mindestens 24 Stunden kontinuierlich freizusetzen und beim Patienten zu einem Plasmaspiegel von zwischen 0.1 und 15 na Rotigotin/ml zu führen.

In einer bevorzugten Ausführungsform ist die pharmazeutische Formulierung als ölige Suspension ausgebildet, wobei das Rotigotin in fester Form vorliegt.

In einer anderen bevorzugten Ausführungsform liegt Rotigotin Salz in kristalliner Form in einer wasserfreien Formulierung vor, die nach Applikation bei einem Säuger zu einem kontinuierlichen Plasmaspiegel von 0,2-10 ng Rotigotin/ml Blut über mindestens 24 Stunden führt.

Überraschenderweise gelingt es mit dieser sehr einfach aufgebauten erfindungsgemäßen Formulierung einen über 48 Stunden therapeutisch relevanten Plasmaspiegel von Rotigotin zu erreichen. Dabei ist die erfindungsgemäße Zusammensetzung einfach und kostengünstig herzustellen, bioabbaubar, untoxisch und gut verträglich.

Weiterhin enthält die erfindungsgemäße pharmazeutische Zusammensetzung in besonders vorteilhafterweise nur wenige, gut charakterisierte und gut verträgliche Hilfsstoffe.

Durch individuelle Einstellung des Applikationsvolumens oder der Wirkstoffkonzentration ist zudem sehr leicht möglich, die Dosierung an die individuellen Bedürfnisse, Symptomatik und Verfassung des jeweiligen Patienten anzupassen.

Die erfindungsgemäßen therapeutischen Zusammensetzungen sind daher ausgezeichnet zur Behandlung von Erkrankungen mit Dopamin-Stoffwechseistörungen wie Morbus Parkinson oder Restless Leg geeignet. Die Therapie kann dabei sowohl als Monotherapie als auch in Kombination mit anderen Wirkstoffen erfolgen.

Schließlich zeigte sich völlig unerwarteterweise, dass Rotigotin beim Erhitzen während des Hitze-Sterilisationsprozesses stabil bleibt, wenn es in fester Form in den erfindungsgemäßen pharmazeutischen Zusammensetzungen vorliegt. Demgegenüber wird Rotigotin in gelöster Form während der Hitzesterilisation einem erheblichen thermischen Abbau unterworfen.

### Abbildungen:

Abbildung 1 zeigt Plasmakonzentrationen von Rotigotin nach subkutaner Verabreichung vier verschiedener Dosen Rotigotin in öliger Kristallsuspension in der Ratte. Die Verabreichung erfolgte alle 48 Stunden über mehrere Wochen. Abbildung 1 A zeigt gemittelte Meßwerte nach der 2. Applikation, Abbildung 1 B nach der 46. Applikation.

Abbildung 2 zeigt Plasmakonzentrationen von Rotigotin nach subkutaner Verabreichung von 12,5 mg Rotigotin pro kg Körpergewicht in der Ratte. Die Verabreichung erfolgte alle 48 Stunden. Wiedergegeben wurden Plasmaspiegel einzelner Tiere jeweils 2, 4, 8, 24, 32 und 48 Stunden nach der 22. Applikation.

Abbildung 3 zeigt Plasmakonzentrationen von Rotigotin nach 85maliger Applikation von 1 mg/kg Rotigotin (Abb. 3A) und 4 mg/kg Rotigotin (Abb. 3B) in Form öliger Rotigotin Kristallsuspension im Affen.

Abbildung 4 zeigt die Korrelation zwischen applizierter Dosis Rotigotin in Form öliger Kristallsuspensionen und dem maximalen Plasmaspiegel nach 3maliger und 85maliger täglicher Applikation im Affen.

### Beschreibung der Erfindung:

Die vorliegende Erfindung betrifft pharmazeutische Zusammensetzungen umfassend Rotigotin als Wirkstoff, wobei die pharmazeutische Zusammensetzung als injizierbares Depot ausgebildet ist.

Unter dem Ausdruck "Depotform" oder "Depot" wird in dieser Patentanmeldung eine nichttransdermale pharmazeutische Formulierung verstanden, die nach Applikation beim Patienten zu einem therapeutisch effektiven Rotigotin Plasmaspiegel über einen Zeitraum von mindestens 24 Stunden führt. Bevorzugte Rotigotin-Plasmaspiegel liegen zwischen 0,2 und 10 ng/ml, insbesondere zwischen 0,3 und 5 ng/ml und ganz besonders zwischen 0,4 und 3 ng/ml.

Unter dem Ausdruck "Rotigotin" wird die Substanz 2-(N-propyl-N-2-thienylethylamino)-5-hydroxytetralin sowie pharmazeutisch akkzeptable Salze davon verstanden.

Unter dem Ausdruck "Rotigotin Derivate" werden substituierte 2-Aminotetraline verstanden, wie in US-Patent US 4,564,628 beansprucht.

Beispiele für Depotformen sind z.B. Mikropartikel oder Mikrokapseln; lipidbasierte Nanopartikel; Komplexe des Wirkstoffs mit oder Einbettung in organische oder anorganische Stoffe wie z.B. Gelatine, Polyvinylpyrrolidon, Carboxymethylcellulose oder Polyglutaminsäure; Emulsionen oder Suspensionen.

Die Herstellung geeigneter Mikrokapseln oder Mikropartikel kann in an sich bekannter Weise erfolgen, z.B. durch Mikroverkapselung oder Sprühtrocknung auf Basis bioabbaubarer Polymere wie Polylactid-Polyglycolid Copolymere (PLGA), wie beispielsweise in EP 0 625 069 beschrieben.

In einer bevorzugten Ausführungsform ist die Depotform als Suspension, vorzugsweise als ölige Suspension, ausgebildet. In dieser Suspension liegt das Rotigotin im wesentlichen in fester Phase vor, die in einem flüssigen Vehikel suspendiert ist.

Unter dem Ausdruck "im wesentlichen" wird in dieser Patentanmeldung über 90% verstanden.

Bevorzugt werden pharmazeutische Zusammensetzungen, in denen Rotigotin zu über 95%, besonders bevorzugt zu über 97% oder zu über 99% in fester Phase vorliegt.

Unter dem Ausdruck "ölige Suspension" wird in dieser Patentanmeldung ein disperses System verstanden, in dem die kontinuierliche Phase (das "Vehikel") in Form eines flüssigen Lipids vorliegt.

Unter dem Ausdruck "feste Phase" wird in dieser Patentanmeldung das Vorliegen von Rotigotin in fester Form verstanden. Dabei kann Rotigotin in freier fester Form z.B. als Kristalle oder als amorphe Partikel vorliegen oder aber auch an ein geeignetes Trägermaterial, z.B. an PLGA-Mikropartikel, gebunden sein.

Ein bevorzugter Gegenstand der Erfindung sind pharmazeutische Formulierungen, in denen Rotigotin weitgehend unlöslich ist. Dies führt zu einer protahierten Freisetzung von Rotigotin aus der festen Phase nach Verabreichung der Formulierung an einem lebenden Organismus, z.B. in Form eines Unterhautdepots.

Unter dem Ausdruck "in der pharmazeutischen Formulierung weitgehend unlöslich" wird verstanden, dass bei Raumtemperatur weniger als 10% des eingesetzten therapeutischen Wirkstoffs in der pharmazeutischen Formulierung in gelöster Form vorliegt.

Besonders bevorzugt werden Formulierungen, in denen Rotigotin zu weniger als 5%, besonders bevorzugt weniger als 3% oder ganz besonders bevorzugt zu weniger als 1% löslich sind.

Ein bevorzugter Gegenstand der Erfindung sind daher pharmazeutische Zusammensetzungen umfassend Rotigotin, die wasserfrei sind.

Unter dem Ausdruck "wasserfrei" wird in dieser Patentanmeldung ein Wasseranteil unter 3% verstanden.

Besonders bevorzugte Formulierungen haben einen Wasseranteil von unter 1% oder ganz besonders bevorzugt von unter 0,5 %.

Aus der festen Phase wird der Wirkstoff nach Applikation über einen längeren Zeitraum kontinuierlich freigesetzt, so daß trotz der schnellen biologischen Elimination von Rotigotin ein therapeutisch effektiver Plasmaspiegel von 0,1-15 ng/ml über einen Zeitraum von mindestens 24 Stunden, bevorzugt von mehr als 36 Stunden, besonders bevorzugt mehr als 48 Stunden erreicht wird. Bevorzugt werden Plasmaspiegel von 0,2 - 10 ng Rotigotin/ml, besonders bevorzugt von 0,3 - 5 ng/ml und ganz besonders bevorzugt von 0,4 - 3 ng/ml eingestellt.

Vorteilhafterweise kann dadurch die Verabreichungsfrequenz von Rotigotin auf eine einzelne Applikation pro Tag oder eine Verabreichung alle zwei oder drei Tage gesenkt werden.

Ein Gegenstand der Erfindung ist daher eine pharmazeutische Formulierung zur Verabreichung von Rotigotin über einen Zeitraum von mindestens 24 Stunden, bevorzugt mindestens 36 oder 48 Stunden.

Dies kann erreicht werden, indem Rotigotin in ein gut wasserlösliches, pharmazeutisch akzeptables Salz überführt wird, das keine oder nur schwache Löslichkeit in aliphatischen Lösungsmitteln, insbesondere in Ölen zeigt und das somit in einer entsprechenden wasserfreien Formulierung weitgehend unlöslich ist.

Beispiele für solche pharmazeutisch akzeptablen Salze sind z.B. Salze anorganischer oder organischer Säuren, wie z.B. Hydrochlorid, Hydrobromid, Hydrogensulfat, Carbonsäuren oder Alkansulfonsäuren oder Salze mit Metallkationen.

Ein besonders bevorzugtes Beispiel ist Rotigotin Hydrochlorid.

Weniger geeignet ist hingegen die freie Base von Rotigotin, die vergleichsweise gut in organischen Lösungsmitteln und aliphatischen Kohlenwasserstoffen löslich ist.

Bevorzugt werden pharmazeutische Zusammensetzungen, in denen Rotigotin im wesentlichen in Form freier Kristalle oder amorpher Partikel vorliegt.

Besonders bevorzugt werden pharmazeutische Depotformen, die Rotigotin-Kristalle enthalten.

Rotigotin Kristalle können in einfacher Weise durch Umkristallisierung eines entsprechenden Rotigotin Salzes mit organischen Lösungsmitteln hergestellt werden, wie es beispielsweise in der USP 4,564,628 beschrieben ist.

Kristallines Rotigotin Hydrochlorid kann beispielsweise hergestellt werden, indem Rotigotin Hydrochlorid zunächst unter Wärme in Methanol gelöst wird, das Methanol abdestilliert wird, der Rückstand in Aceton bei über 50°C gelöst wird und das Rotigotin-HCl sodann für mehrere Stunden bei niedrigen Temperaturen auskristallisiert wird. Zur weiteren Aufreinigung kann eine Rekristallisation z.B. in Aceton oder Propanol erfolgen.

Die kristallinen, hydrophilen Rotigotin Salze werden schließlich in wasserfreie injizierbare Formulierungen eingebracht, in denen besagte Salze schwer löslich oder weitgehend unlöslich sind.

Solche pharmazeutische Formulierungen können beispielsweise auf einer kontinuierlichen Phase beruhen, die aus pharmazeutisch akzeptablen, flüssigen Glycerinfettsäureestern, Fettalkoholen, aliphatischen Kohlenwasserstoffen (z.B. Paraffin) oder hydrophoben flüssigen Silikonen besteht und in die Rotigotin Salz, z.B. Rotigotin Hydrochlorid, in kristalliner Form eingebracht ist. Aus dieser lipidhaltigen bzw. hydrophoben Phase wird das hydrophile kristalline Rotigotin Salz nach Applikation beim Patienten nur langsam und somit retardiert freigesetzt.

In einer bevorzugten Ausführungsform betrifft die Erfindung daher eine wasserfreie hydrophobe pharmazeutische Zusammensetzung, die festes, bevorzugt kristallines Rotigatin-Salz enthält und die bei Applikationsintervallen von mindestens 24 Stunden die Einstellung eines kontinuierlichen Plasmaspiegels von 0,2 ng - 10 ng Rotigotin/ml über den gesamten Verabreichungszeitraum erlaubt.

Die verabreichte Dosis Rotigotin und damit der Plasmaspiegel lässt sich zum einen durch die Konzentration des Wirkstoffs in der Formulierung und zum anderen durch die Wahl des Injektionsvolumens steuern.

So kann das Volumen in einem weiten Bereich von 5-1500 µl variiert werden, so daß die Dosierung in besonders einfacher Weise auf die individuelle Situation des Patienten einstellbar ist.

Bevorzugte Applikationsvolumina sind Volumina zwischen 10 und 500 µl oder zwischen 100 und 1000 µl.

Der Konzentrationsbereich für Rotigotin wird hauptsächlich durch die pharmakologischen Wirkungen von Rotigotin nach Applikation des Depots bestimmt. Ein geeigneter Konzentrationsbereich für Rotigotin ist 0.01-10% (w/v), bevorzugt 0.02-5%, besonders bevorzugt ist eine Konzentration von 0.1-2%.

Geeignete Tagesdosen Rotigotin sind z.B. 0,5-40 mg, bevorzugt 1-20 mg, besonders bevorzugt 2-15 mg, ganz besonders bevorzugt 2-10 mg.

In einer Ausführungsform ist die pharmazeutische Zusammensetzung als Rotigotin in fester Phase enthaltende ölige Suspension ausgebildet, deren kontinuierliche Phase ein Lipid ist.

Pharmazeutisch einsetzbare Lipide sind z.B. pflanzliche Öle wie Mandelöl, Olivenöl, Mohnöl, Erdnussöl oder Sesamöl, höhere Fettsäuren wie Ölsäure, sowie Mono-, Di- oder Trifettsäureester von Mono- oder Polyolen wie z.B. Isopropanol, 1,3-Propandiol, Glycerol, 1,2-Butandiol oder 1,2,3-Butandiol.

In einer bevorzugten Ausführungsform ist die pharmazeutische Zubereitung eine ölige Suspension, deren Vehikel im wesentlichen aus Polyol-Fettsäure-Estern besteht.

Unter dem Ausdruck "Polyol-Fettsäure-Ester" werden in dieser Patentanmeldung auch Gemische verschiedener Polyol-Fettsäure-Ester subsumiert.

Als Polyol-Komponente besagter Polyol-Fettsäure-Estern werden Polyole mit zwei bis vier C-Atomen und einer variablen Anzahl an Hydroxygruppen bevorzugt. Geeignete Beispiele sind 1,3-Propandiol, Glycerol, 1,2,3-Butantriol, 1,2,4-Butantriol oder 1,3-Butandiol.

Besonders bevorzugt werden Glycerol, 1,3-Propandiol und/oder 1,3-Butandiol als Polyol-Komponente der kontinuierlichen Phase.

Der Gesamt-Veresterungsgrad der Polyol-Fettsäure-Ester der kontinuierlichen Phase beträgt bevorzugt 80-100%, besonders bevorzugt 90-100%.

Vorzugsweise liegt die Kettenlänge der Fettsäuren in besagten Polyol-Fettsäure-Estern des Vehikels zwischen 6 und 22 C-Atomen und besonders bevorzugt zwischen 6 und 14 C-Atomen.

Das Vehikel beeinhaltet dabei vorzugsweise über 60% gesättigte Fettsäuren. Besonders bevorzugt enthält das Vehikel über 90% gesättigte Fettsäuren.

Als wesentliches Bestandteil des Vehikels ganz besonders bevorzugt werden mittelkettige Triglyceride (MKTs), die hauptsächlich gesättigte Fettsäuren mit Kettenlängen von 8-10 C-Atomen enthalten und die in Pharmakopöen beschrieben sind.

Ein bevorzugter Gegenstand der Erfindung sind daher pharmazeutische Depotformen von Rotigotin, die als ölige Suspension ausgebildet sind und ein Vehikel umfassen, das im wesentlichen aus MKTs besteht.

MKTs sind gut charakterisierte Substanzen, die sich für systemische Verabreichungsformen bewährt haben. Vorteilhafterweise sind MKTs bioabbaubar, nicht reizend und haben exzellente physikochemische Eigenschaften für die Verwendung auch in injizierbaren Arzneiformen. MKTs eignen sich daher ganz besonders als Vehikel für die erfindungsgemäßen pharmazeutischen Zusammensetzungen.

Beispielhaft genannt sei ein kommerziell erhältlicher Triglycerid-Caprylsäure/Caprinsäureester, der unter dem Handelsnamen Miglyol 812^{R} (Fa. Condea) erhältlich ist.

Der Anteil der kontinuierlichen Phase (des Vehikels) an der pharmazeutischen Zusammensetzung ergibt sich aus den Konzentrationen von Wirkstoff, Netzmittel und etwaigen übrigen Hilfsstoffen und ist üblicherweise über 75%, bevorzugt 88-99.8%; ganz besonders geeignet ist eine Konzentration von 94-99%.

In einer bevorzugten Ausführungsform der Erfindung ist die pharmazeutische Zusammensetzung eine ölige Suspension, die ferner ein Netzmittel enthält. Unter dem Ausdruck "Netzmittel" wird dabei ein Stoff verstanden, der die Grenzflächenspannung zwischen Vehikel- und Wirkstoffoberfläche herabsetzt.

Netzmittel sind dem Fachmann aus dem Stand der Technik bekannt. Als den Schutzbereich nicht einschränkende Beispiele seien an dieser Stelle folgende Netzmittel genannt:
Kondensationsprodukte aus Polyolen und Carbonsäuren, wie z.B. Fettsäureester von Isopropanol, Glycerol, 1,3-Butandiol, 1,2,4-Butantriol, 1,2,3-Butantriol, 1,3-Propandiol, Saccharose, Sorbitan, Propylenglycol, Polyoxyethylen, Polyoxyethylensorbitol oder Dextrin.
Kondensationsprodukte aus Polyolen und langkettigen Alkoholen wie z.B. Polyoxyethylencetanol oder Polyoxypropylenhexadecanol.

Bevorzugte Netzmittel für die erfindungsgemäßen pharmazeutischen Zusammensetzungen bestehen im wesentlichen aus Polyol-Fettsäure-Estern.

In einer bevorzugten Ausführungsform enthält die erfindungsgemäße pharmazeutische Zusammensetzung als Netzmittel im wesentlichen Polyol-Fettsäure-Ester mit einem Monoester-Anteil von über 60%, bevorzugt von über 90%.

Diese Polyol-Fettsäure-Ester enthalten bevorzugt Polyole mit zwei bis sechs C-Atomen, wie z.B. Glycerol, 1,3-Butandiol, 1,3-Propandiol, 1,2,3-Butantriol, 1,2,4-Butantriol, Isopropanol, Saccharose oder Sorbitan.

Besonders bevorzugt werden als Netzmittel für die erfindungsgemäßen pharmazeutischen Zusammensetzungen Polyol-Fettsäure-Ester, die Glycerol oder 1,2,3-Butantriol enthalten.

Die bevorzugte Kettenlänge der Fettsäuren in besagten Polyol-Fettsäure-Estern des Netzmittels ist von 6 bis 22 C-Atomen, besonders bevorzugt von 6 bis 14 C-Atomen.

Die Polyol-Fettsäure-Monoester des Netzmittels beeinhalten dabei vorzugsweise zu über 60% gesättigte Fettsäuren. Besonders bevorzugt enthalten die Polyol-Fettsäure-Monoester über 90% gesättigte Fettsäuren.

In einer weiteren bevorzugten Ausführungsform der Erfindung werden als Netzmittel im wesentlichen Glycerol oder 1,2,3-Butantriol verwendet, die mit gesättigten Fettsäuren mit 6-14 C-Atomen verestert sind.

Besonders als Netzmittel bevorzugt werden in Pharmakopoen beschriebene Handelsprodukte verwendet, wie z.B. Glycerolmonocaprylat (Imwitor 308, Fa.Condea).

Ganz besonders bevorzugt werden pharmazeutische Zusammensetzungen, die als Netzmittel Glycerolmonolaurat enthalten, welches z.B. unter dem Handelsnamen Imwitor 312^{R} käuflich erhältlich ist. Glycerolmonolaurat ist eine gut charakterisierte Substanz, die in Deutschland als Nahrungsmittelzusatz zugelassen ist und die sich als besonders geeignet zur Verwendung in den erfindungsgemäßen Depotformen herausgestellt haben.

Ein besonders bevorzugter Gegenstand der Erfindung ist daher eine pharmazeutische Zussammensetzung, die Rotigotin in fester Phase, ein flüssiges Vehikel und ein Netzmittel enthält, wobei das Netzmittel im wesentlichen aus Glycerolmonolaurat und/oder Glycerolmonocaprylat besteht.

In einer weiteren bevorzugten Ausführungsform ist die Rotigotin-haltige wasserfreie pharmazeutische Zusammensetzung frei von Phosphatiden. Überraschenderweise stellten die Erfinder fest, dass der Zusatz des in der Literatur als Netzmittel beschriebenen Lecithins die Retardwirkung der kristallinen Rotigotin enthaltenden Formulierung aufhebt. Ein Aspekt der Erfindung ist daher eine wasserfreie pharmazeutische Retardform, die kristallines Rotigotin-Salz enthält und frei von Lecithin ist.

Der Konzentrationsbereich für das Netzmittel richtet sich nach der Menge des Wirkstoffs. Die Netzmittelkonzentration muß ausreichend sein, um eine Benetzung der Wirkstoffpartikel zu gewährleisten. Dies kann mit geeigneten Tests, die dem Fachmann bekannt sind, in einfacher Weise bestimmt werden. Andererseits muß darauf geachtet werden, dass das gewählte Netzmittel in einer Konzentration eingesetzt wird, in der das Netzmittel noch nicht auskristallisiert.

Ein geeigneter Konzentrationsbereich (w/w) für die Netzmittel ist 0.02-10%, bevorzugt 0.1-5%, besonders bevorzugt ist eine Konzentration von 0.5-2,5%, wobei die Konzentration des Netzmittels jeweils der Konzentration des Wirkstoffs angepasst wird.

Die erfindungsgemäße pharmazeutische Zusammensetzung kann weitere Hilfs- und Zusatzstoffe enthalten, die dem Fachmann auf dem Gebiet der Galenik bekannt sind. So ist der Zusatz lipidlöslicher Antioxidanzien, wie z.B. Vitamin E sinnvoll, wenn besagte Zusammensetzungen z.B. ein Vehikel und/oder Netzmittel umfassen, die ungesättigte Fettsäuren enthalten. Ferner kann die pharmazeutische Zusammensetzung gegebenenfalls Verdickungsmittel enthalten.

In einem anderen Aspekt der Erfindung umfasst die erfindungsgemäße pharmazeutische Zusammensetzung die folgenden Bestandteile:
(a) Rotigotin in fester Phase,
(b) ein Vehikel, im wesentlichen bestehend aus Polyol-Fettsäure-Estern mit einem Gesamt Veresterungsgrad über 80%
(c) ein Netzmittel im wesentlichen bestehend aus Polyol-Fettsäure-Estern mit einem Monoester-Anteil über 60 %.

Hierfür geeignete Vehikel und Netzmittel sind die bereits weiter oben aufgeführten und diskutierten.

Ein weiterer Aspekt der Erfindung ist die Herstellung von Rotigotin Depotformen.

Eine solche Depotform kann in besonders einfacher und vorteilhafter Weise hergestellt werden, indem festes Rotigitin in einer flüssigen öligen Phase suspendiert wird. Als Vehikel der öligen Phase wird dabei ein Polyol-Fettsäure-Ester, bevorzugt ein Triester eines Polyols mit zwei bis vier C-Atomen mit einer Fettsäure einer Kettenlänge von 6 bis 14 C-Atomen, z.B. ein Mittelkettentriglycerid verwendet. Ferner kann als Netzmittel ein Polyol-Monofettsäure-Ester, bevorzugt ein Monoester eines Polyols mit zwei bis sechs C-Atomen mit einer Fettsäure einer Kettenlänge von 6 bis 14 C-Atomen, z.B. Glycerinmonolaurat, zugesetzt werden. In einer bevorzugten Ausführungsform dieses Herstellungsverfahrens wird Rotigotin kristallin verwendet, besonders bevorzugt als kristallines Hydrochloridsalz, und die Zusammensetzung wird ohne Zusatz von Wasser hergestellt.

Sinnvolle Konzentrationen (w/w) für die erfindungsgemäße Herstellung einer Rotigotin-Depotform sind für Rotigotin 0,01-10%, bevorzugt 0,02-5%, besonders bevorzugt 0.1-2%, für das Vehikel 75-99,9%, bevorzugt 88-99,8%, besonders bevorzugt 94-99% und für das Netzmittel 0,02-10%, bevorzugt 0,1-5% und besonders bevorzugt 0,5-2,5%.

Ein weiterer Aspekt der Erfindung ist eine im wesentlichen wasserfreie pharmazeutische Zusammensetzung, die Rotigotin, bevorzugt in kristalliner Form, enthält.

Die erfindungsgemäßen pharmazeutischen Zusammensetzungen sind grundsätzlich geeignet zur mukosalen, z.B. nasalen, oder zur parenteralen Applikation.

Besonders geeignet sind die Zusammensetzungen für die Verabreichung durch Injektion, wobei die Injektion sowohl durch herkömmliche Spritzen als auch durch nadellose Injektionssysteme erfolgen kann. Beispiele für solche nadellosen Injektionssysteme sind in den Patentschriften US 5,840,062 und US 4,913,699 beschrieben. Die Injektion kann auf einem der im Stand der Technik bekannten Applikationswege für Depotformen, wie subkutan, intrakutan, intramuskulär oder intrakranial, z.B. intraventrikulär, erfolgen.

Bevorzugt wird die subkutane, intrakutane oder intramuskuläre Verabreichung, ganz besonders bevorzugt wird die subkutane Applikation.

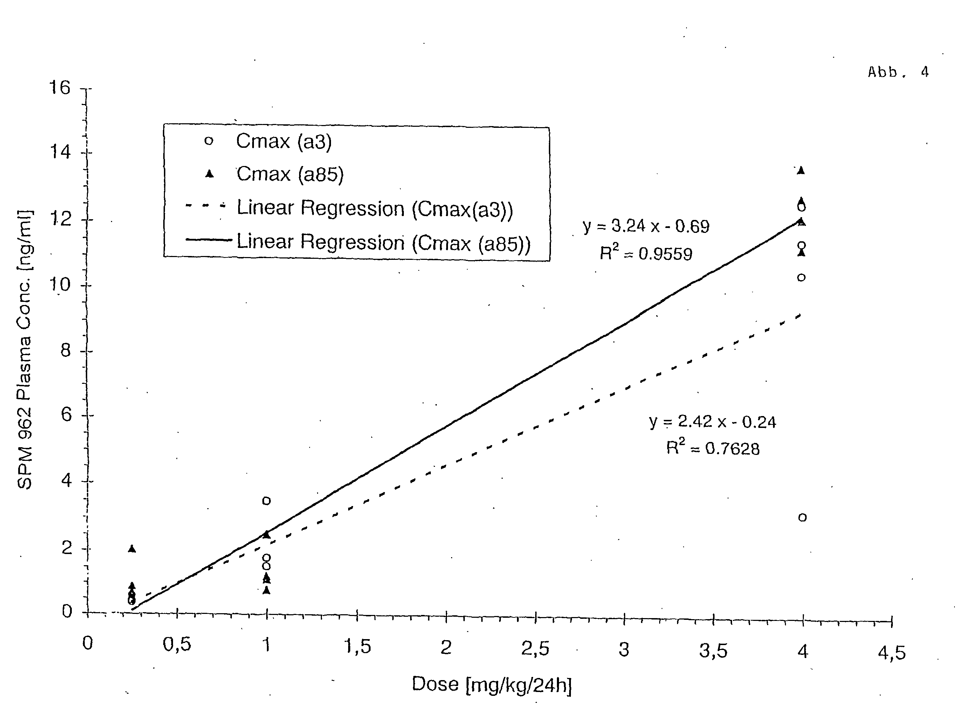

Überraschenderweise gelingt es mit den sehr einfach aufgebauten erfindungsgemäßen Formulierungen einen über 48 Stunden therapeutisch relevanten Plasmaspiegel von Rotigotin zu erreichen. Die Bioverfügbarkeit der erfindungsgemäßen pharmazeutischen Zusammensetzungen beträgt für Rotigotin über 70% und die erreichten Plasmawirkstoffspiegel stehen in weitgehend linearer Beziehung zur in den Körper verbrachten Dosis (siehe Abbildung 4).

Daher sind die erfindungsgemäßen therapeutischen Zusammensetzungen ausgezeichnet zur chronischen Behandlung von Erkrankungen geeignet, die mit einer Störung des Dopamin-Stoffwechsels einhergehen. Beispiele für solche Erkrankungen sind Morbus Parkinson oder das Restless Leg Syndrom.

Die erfindungsgemäßen Formulierungen können dabei als sowohl Monotherapie oder in Kombination mit anderen Antiparkinsonmittel eingesetzt werden.

Unter dem Ausdruck "Antiparkinsonmittel" wird dabei jeder Wirkstoff verstanden, der geeignet ist, einen pathologisch veränderten Dopaminstoffwechsel günstig zu beeinflussen und/oder in anderer Weise das Fortschreiten oder Bestehen von Morbus Parkinson therapeutisch oder prophylaktisch zu vermindern oder zu verhindern und/oder die mit Morbus Parkinson verbundenen Begleitsymptome zu lindern.

Beispiele für solche Antiparkinsonmittel sind dem Fachmann bekannt. Den Schutzbereich nicht-limitierende Beispiele für geeignete weitere Wirkstoffe sind Vertreter der Gruppe metabolischer Dopaminvorstufen, Dopaminrezeptoragonisten, Dopamintransportblocker, MAO-Hemmer, Muskarin-Rezeptorantagonisten, Glutamat-Rezeptorantagonisten, Catechol-O-Methyltransferase-Blocker, Neurotrophine, Immunophilin-Liganden, Histamin-Antagonisten, Antioxidanzien, Glutathion Transferase-Aktivatoren, Anti-Apoptose-Wirkstoffe oder Calciumantagonisten.

Geeignete Vertreter sind insbesondere Levodopa, Methyldopa, Biperiden, Paragylin, Rasagilin, Selegilin, Lisurid, Pergolid, Bromocriptin, Cabergolin, Benzatropin, Ropinirol, A-mantadin, Memantine, Trihexyphenidyl, Diphenhydramin, Dihydroergocryptin, Tolcapon, Entacapon, Metixen, Procyclidin, Budipin, Bornaprin, Pramipexol, Glial cell line-derived neurotrophic factor (GDNF) und der Brain-derived neurotrophic factor (BDNF).

Besagte Wirkstoffe können gemeinsam mit Rotigotin in der erfindungsgemäßen pharmazeutischen Zubereitung verabreicht werden oder aber in einer separaten injizierbaren oder nicht-injizierbaren Formulierung, z.B. als "Kit-of-Parts", vorliegen. Dabei kann der zusätzliche Antiparkinson-Wirkstoff ebenfalls retardiert oder aber unretardiert formuliert sein.

Mit der vorliegenden Erfindung wurden zum erstenmal injizierbare Depotformulierungen von Rotigotin zur Verfügung gestellt, die in der Lage sind, Rotigotin über einen Zeitraum von wenigstens 24 Stunden in therapeutisch wirksamer Menge freizusetzen. Ein weiterer Aspekt der Erfindung ist daher die Verwendung von Rotigotin zur Herstellung eines Depot-Arzneimittels.

Die in der vorliegenden Patentanmeldung beschriebenen erfindungsgemäßen pharmazeutischen Zusammensetzungen sind auch zur Verabreichung von Rotigotin Derivaten geeignet, die in der US-Patentschrift US 4,564,628 beschrieben sind. Ein weiterer Gegenstand der Erfindung sind daher pharmazeutische Depotformen von Rotigotin Derivaten sowie die Verwendung von Rotigotin Derivaten zur Herstellung von Depot-Arzneimitteln.

Ein anderer Aspekt der Erfindung ist die Verwendung von Rotigotin in fester Phase zur Herstellung eines hitzesterilisierbaren Arzneimittels. Es zeigte sich unerwarteterweise, dass Rotigotin beim Erhitzen während des Sterilisationsprozesses stabil bleibt, wenn es in fester Phase vorliegt. Demgegenüber ist gelöstes Rotigotin während des Autoklavierens einem signifikanten thermischen Abbau unterworfen (siehe Ausführungsbeispiel 4).

Ein bevorzugter Gegenstand der Erfindung ist die Verwendung von kristallinem Rotigotin, insbesondere als Hydrochloridsalz, zur Herstellung eines hitzesterilisierbaren Arzneimittels.

Unter dem Ausdruck "hitzesterilisierbares Arzneimittel" wird in dieser Patentanmeldung verstanden, das beim Erhitzen der pharmazeutischen Formulierung auf 121 °C und 0,2 mPa über 20 Minuten weniger als 1% des Wirkstoffs abgebaut wird.

Ein weiterer Gegenstand der Erfindung ist die Herstellung einer sterilen pharmazeutischen Zusammensetzung enthaltend Rotigotin durch das Autoklavieren einer pharmazeutischen Formulierung, die Rotigotin in fester Phase enthält. Ein Beispiel ist das Autoklavieren einer erfindungsgemäßen pharmazeutischen Formulierung bei 121 °C und 0.2 MPa über 20 Minuten.

In einer bevorzugten Ausführungsform ist das Rotigotin dabei als kristallines Hydrochloridsalz ausgebildet, welches z.B. in öliger Suspension vorliegt.

Ein anderer Aspekt der Erfindung ist die Bereitstellung eines Kits zur Behandlung von Morbus Parkinson oder Restless Leg umfassend eine erfindungsgemäße pharmazeutische Zusammensetzung und eine Vorrichtung zur Injektion.

Bei der Vorrichtung zur Injektion kann es sich um ein noch mit der pharmazeutischen Zusammensetzung zu beschickendes oder um ein bereits mit der erfindungsgemäßen pharmazeutischen Zubereitung befülltes Injektionssystem handeln. Das Injektionssystem kann mit einer herkömmlichen Kanüle versehen sein oder alternativ auch als nadelloses Injektionssystem zu gebrauchen sein.

Ein Gegenstand der Erfindung ist auch ein Kit in dem mehrere Dosierungen der erfindungsgemäßen pharmazeutischen Zusammensetzung und mehrere Injektionsvorrichtungen enthalten sind, z.B. ein Wochen- oder Monatsvorrat.

Ein weiterer Aspekt der Erfindung ist ein Kit umfassend eine erfindungsgemäße pharmazeutische Zusammensetzung und orale oder transdermale Verabreichungsform von Rotigotin oder eines anderen Antiparkinsonmittels.

Eine Kombination aus injizierbarer und oraler Verabreichungsform in einem Kit kann beispielsweise günstig sein, um ein zu starkes Absinken der Plasmaspiegel bei der Erschöpfung eines Depots und vor dem Applizieren eines neues Depots zu verhindern oder zu überbrücken.

Eine bevorzugte Ausführungsform ist daher ein Kit aus einer injizierbaren Depotform von Rotigotin und einer oralen, schnell anflutenden Formulierung eines Antiparkinsonmittels. Ein Beispiel für schnell anflutende orale Dosierungsformen wird beispielsweise in der EP 651 997 beschrieben.

Ein bevorzugter Gegenstand der Erfindung ist weiterhin ein Kit umfassend die erfindungsgemäße pharmazeutische Zubereitung von Rotigotin und eine transdermale Verabreichungsform von Rotigotin.

Die folgenden Beispiele dienen der Illustration der Erfindung:

### Ausführungsbeispiele:

### 1. Herstellung und Kristallisierung von Rotigotin

Die Herstellung und Kristallisierung von kristallinem Rotigotin erfolgt wie in USP 4,564,628 beschrieben. Alternativ kann kristallines Rotigotin hergestellt werden, indem Rotigotin Hydrochlorid zunächst unter Wärme in Methanol gelöst wird, das Methanol abdestilliert wird, der Rückstand in Aceton bei über 50°C gelöst wird und das Rotigotin-HCl sodann für mehrere Stunden bei niedrigen Temperaturen auskristallisiert wird. Zur weiteren Aufreinigung kann eine Rekristallisation z.B. in Aceton oder Propanol erfolgen.

### 2. Herstellung einer Rotigotin Suspension enthaltend 1% Rotigotin und 1% GML

### (a) Herstellen der kontinuierlichen Phase

1411,2 g Miglyol 812 wurde in eine Duran Flasche eingewogen. 14,4 g Imwitor 312 wurde dem Miglyol zugegeben und im Anschluß für 30 Minuten unter Rühren auf 80°C erwärmt. Die klare Lösung wurde auf Raumtemperatur abgekühlt und gefiltert.

### (b) Herstellen der Suspension

1188 g der unter (b) hergestellten Lösung wurde in einen Glaslaborreaktor überführt, 12 g Rotigotin zugesetzt und für 10 Minuten mit einem Ultraturrax bei 10.000 UpM unter Stickstoff homogenisiert. Die Suspension wurde bei laufendem Ultraturrax (2.000 UpM) in Braunglasflaschen abgefüllt.

### 3. Herstellung einer Rotigotin Suspension enthaltend 0,5, 1,5 und 2% Rotigotin und 0,5%. 1% oder 1,5% GML

Die Herstellung erfolgte wie unter 1 beschrieben, mit entsprechend veränderten Einwaagen.

### 4. Hitzesterilisierundvon Rotigotin

Eine 0,6 %ige wässrige Lösung von Rotigotin Hydrochlorid (Ansatz I) und eine 1%ige Rotigotin Suspension entsprechend Ausführungsbeispiel 2 (Ansatz II) wurde für 20 Minuten bei 120 °C und 0,2 Pa autoklaviert. Zudem wurde eine 0,5%ige wässrige Rotigotin Lösung unter Stickstoffbegasung autoklaviert (Ansatz III). Anschließend wurden photometrisch die Abbaumengen bestimmt.

Beim Autoklavieren der wässrigen Lösungen in den Ansätzen I und III zeigte sich, dass jeweils 1,5 % des Rotigotin thermisch in Abbauprodukte zersetzt wird. In Ansatz II wurden dagegen weniger als 0,5% Abbau nachgewiesen.

### 5. Freisetzung von Rotigotin aus den erfindungsgemäßen Depots in der Ratte

Sprague-Dawley Ratten erhielten subkutane Bolus-Injektionen öliger Rotigotin Kristallsuspension der folgenden Zusammensetzung:

| | |
|---|---|
| Rotigotin | 0,5 oder 1 %ig |
| Imwitor 312 | 1 % |
| Miglyol 812 | ad 100 % |

Die Applikation erfolgte alle 48 Stunden in den folgenden Dosierungen:
1 mg/kg (0,2 ml/kg einer 0,5 %igen Suspension)
3 mg/kg (0,6 ml/kg einer 0,5 %igen Suspension)
10 mg/kg (1 ml/kg einer 1 %igen Suspension)
30 mg/kg (3 ml/kg einer 1 %igen Suspension)

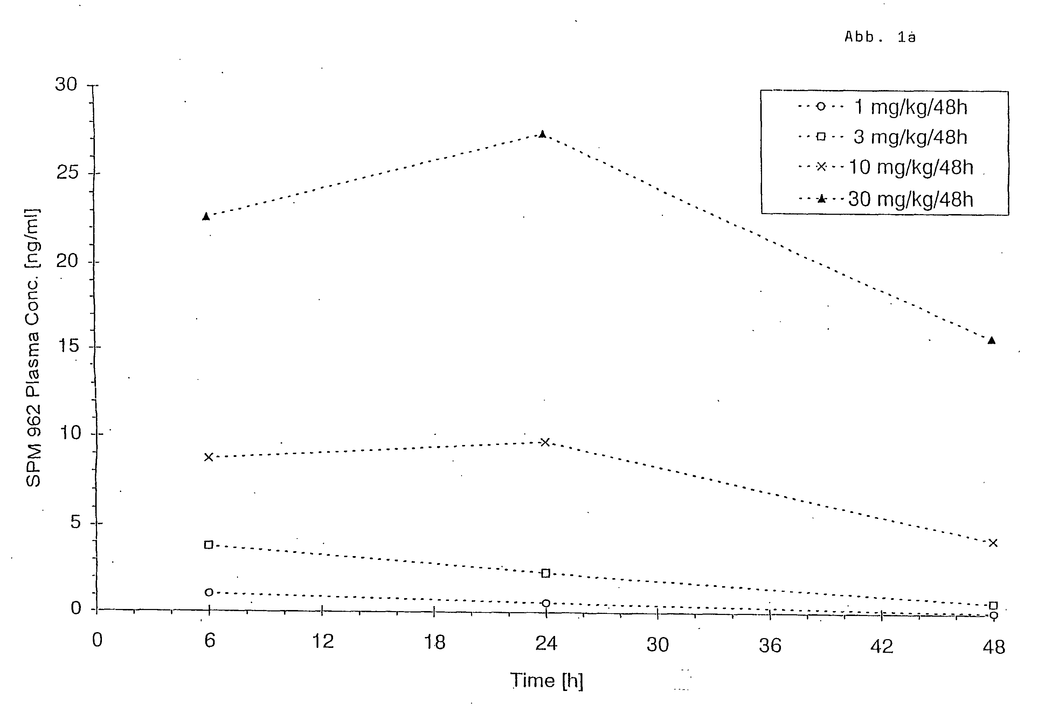

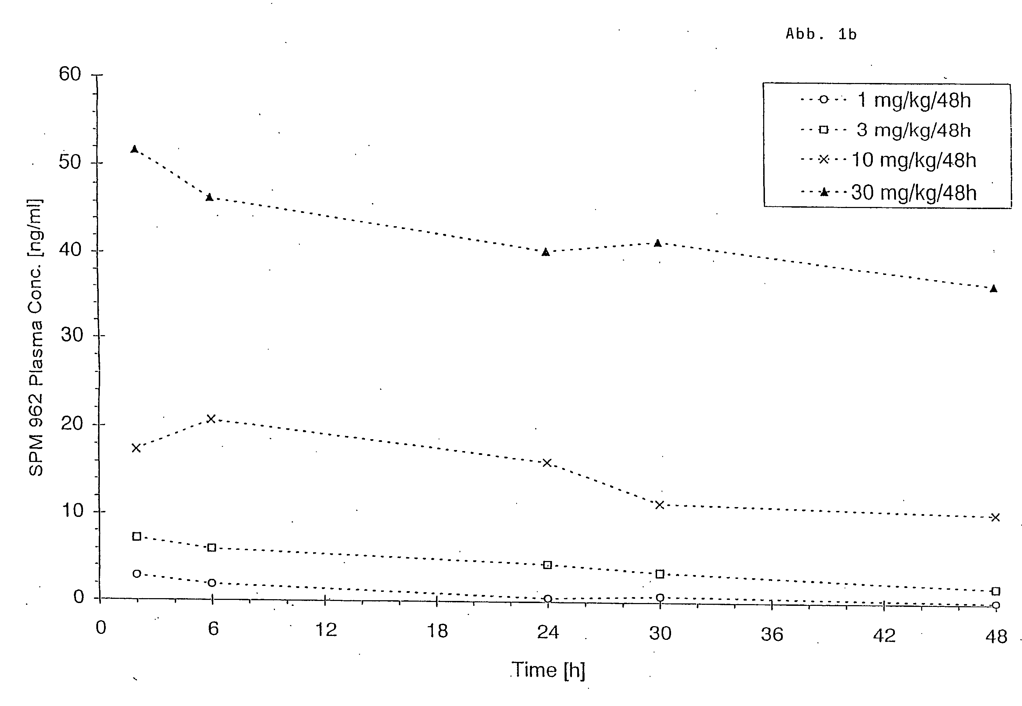

6, 24 und 48 Stunden nach der 2. und 46. Verabreichung wurden Plasmaproben entnommen und die Rotigotin Konzentration mittels LC-MS-MS analysiert. Die Werte aus 6 Tieren wurden gemittelt. Die Resultate sind in Abbildung 1 dargestellt.

### 6. Freisetzung von Rotigotin aus den erfindungsgemäßen Depots in der Ratte

Die Versuchsanordnung entspricht Ausführungsbeispiel 5, mit dem Unterschied, daß alle 48 Stunden eine Dosis von 12,5 mg Rotigotin/kg Körpergewicht appliziert wurde.

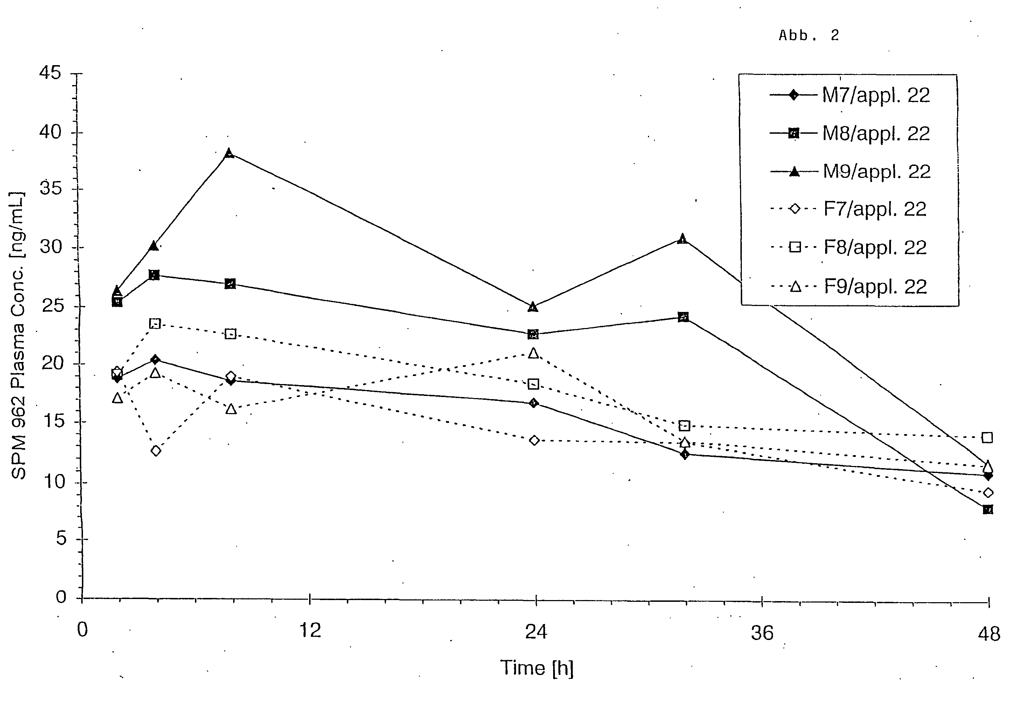

Meßwerte wurden jeweils 2, 4, 8, 24, 32 und 48 Stunden nach der 22. Applikation genommen und quantifiziert. Die Plasmaspiegel der einzelnen Tiere sind in Abbildung 2 dargestellt.

### 7. Freisetzung von Rotigotin aus den erfindungsgemäßen Depots im Affen

Cynomolgus-Affen erhielten tägliche subkutane Bolus-Injektionen öliger Rotigotin Kristallsuspensionen der folgenden Zusammensetzung:

| | |
|---|---|
| Rotigotin: | 0,5 oder 1 %ig |
| Imwitor 312: | 1 %ig |
| Miglyol 812: | ad 100 % |

Die Applikation erfolgte täglich in Dosen von 0,25, 1 und 4 mg/kg. 2, 6 und 24 Stunden nach der 3. und 85. Applikation wurden Plasmaproben entnommen und per LC-MS-MS analysiert.

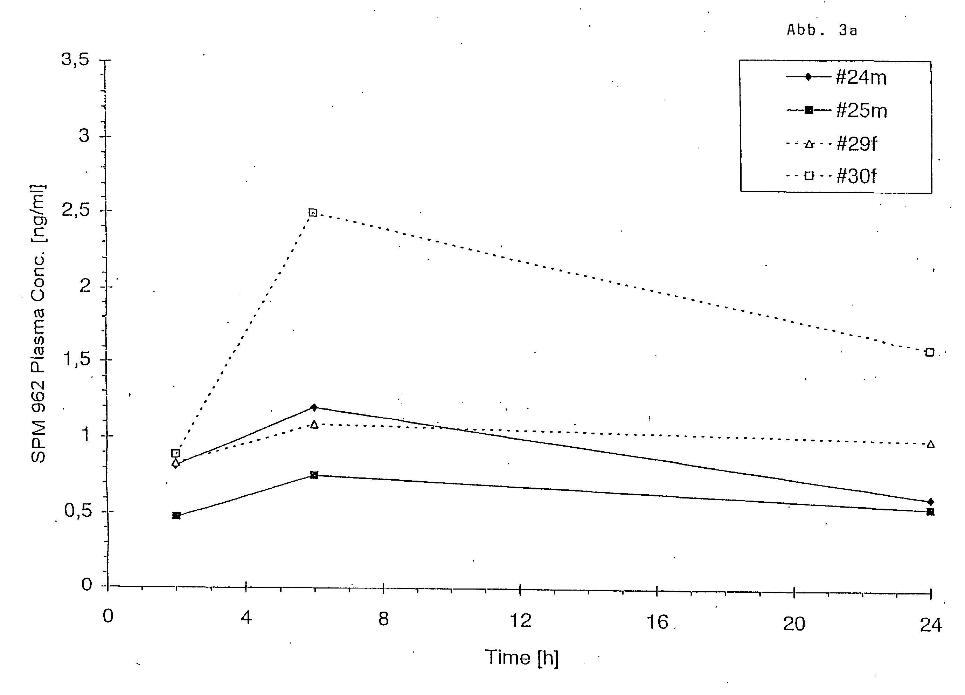

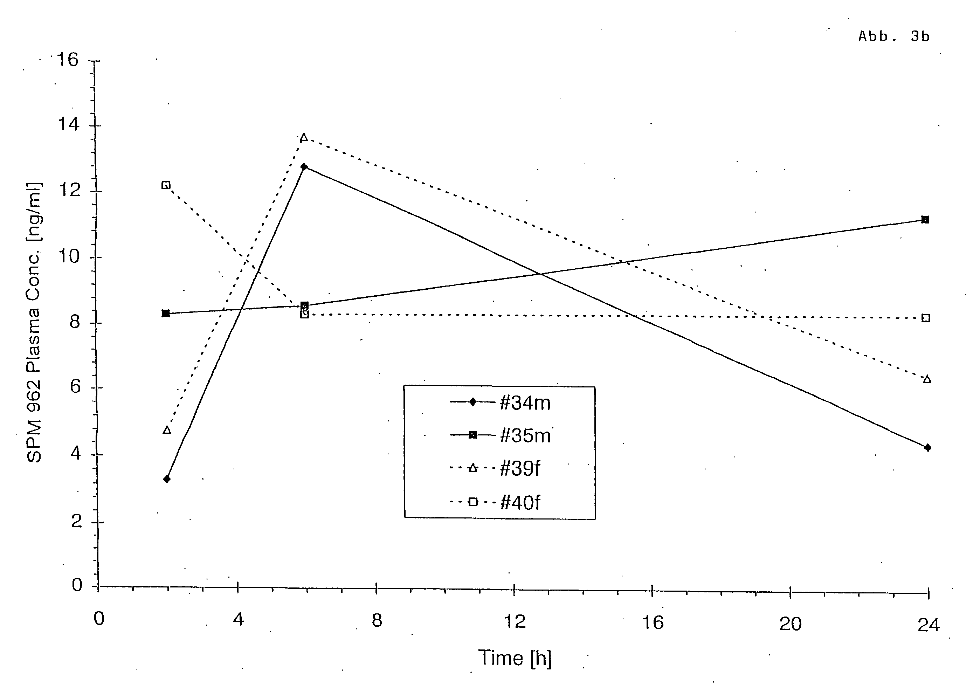

Abbildung 3 zeigt die Meßwerte in einzelnen Tieren. Abbildung 4 zeigt die Beziehung zwischen eingesetzter Dosis und resultierenden maximalen Plasmakonzentrationen.

## Patentansprüche

1. Pharmazeutische Zusammensetzung, **dadurch gekennzeichnet, dass** sie eine den Wirkstoff Rotigotin enthaltende injizierbare Depotform ist, die nach Applikation beim Patienten zu einem Rotigotin-Plasmaspiegel zwischen 0,1 und 15 ng/ml über einen Zeitraum von mindestens 24 Stunden führt.

2. Pharmazeutische Zusammensetzung nach Anspruch 1, **dadurch gekennzeichnet, daß** sie eine Suspension ist, in der der Wirkstoff Rotigotin im wesentlichen in fester Phase vorliegt, die in einem flüssigen Vehikel suspendiert ist.

3. Pharmazeutische Zusammensetzung nach einem der vorhergehenden Ansprüche, **dadurch gekennzeichnet, daß** die pharmazeutische Zusammensetzung wasserfrei ist.

4. Pharmazeutische Zusammensetzung nach einem der vorhergehenden Ansprüche, wobei Rotigotin in kristalliner Form vorliegt.

5. Pharmazeutische Zusammensetzung nach einem der vorhergehenden Ansprüche, wobei Rotigotin als kristallines Salz vorliegt.

6. Pharmazeutische Zusammensetzung nach einem der vorhergehenden Ansprüche, **dadurch gekennzeichnet, daß** sie eine ölige Suspension ist.

7. Pharmazeutische Zusammensetzung nach einem der vorhergehenden Ansprüche, wobei die pharmazeutische Zusammensetzung eine ölige Suspension ist, deren kontinuierliche Phase im wesentlichen aus Polyol-Fettsäure-Estern besteht.

8. Pharmazeutische Zusammensetzung nach Ansprüchen 6-7, wobei die kontinuierliche Phase im wesentlichen aus Polyol-Fettsäure-Estern besteht, die Fettsäuren einer Kettenlänge von 6 bis 22 C-Atomen enthalten.

9. Pharmazeutische Zusammensetzung nach Ansprüchen 6-8, wobei die kontinuierliche Phase im wesentlichen aus Mittelketten-Triglyceriden besteht.

10. Pharmazeutische Zusammensetzung nach Ansprüchen 6-9, ferner **dadurch gekennzeichnet, dass** die pharmazeutische Zusammensetzung zusätzlich ein Netzmittel umfasst.

11. Pharmazeutische Zusammensetzung nach Anspruch 10, wobei das Netzmittel im wesentlichen aus Polyol-Fettsäure-Estern besteht, deren Monoester-Anteil über 60% beträgt.

12. Pharmazeutische Zusammensetzung nach Ansprüchen 10-11, wobei das Netzmittel im wesentlichen aus Polyol-Fettsäure-Monoestern besteht, die Fettsäuren einer Kettenlänge von 6 bis 14 C-Atomen enthalten.

13. Pharmazeutische Zusammensetzung nach Ansprüchen 10-12, wobei das Netzmittel im wesentlichen aus Glycerinmonolaurat und/oder Glycerinmonocaprylat besteht.

14. Injizierbare wasserfreie pharmazeutische Zusammensetzung, **dadurch gekennzeichnet, daß** sie kristallines Rotigotin Salz umfasst, das in hydrophober flüssiger Phase suspendiert ist und nach Verabreichung beim Patienten zu einem kontinuierlichen Plasmaspiegel von 0,2 - 10 ng Rotigotin /ml über mindestens 24 Stunden führt.

15. Pharmazeutische Zusammensetzung nach einem der vorhergehenden Ansprüche zur subkutanen oder intramuskulären Verabreichung.

16. Pharmazeutische Zusammensetzung nach einem der vorhergehenden Ansprüche zur Behandlung von Krankheiten, die mit einem gestörten Dopamin-Stoffwechsel verbunden sind.

17. Pharmazeutische Zusammensetzung nach einem der vorhergehenden Ansprüche ferner **dadurch gekennzeichnet dass** die pharmazeutische Zusammensetzung mindestens einen weiteren Antiparkinsonwirkstoff enthält.

18. Verwendung von Rotigotin oder eines Rotigotin-Derivats zur Herstellung eines injizierbaren Depot-Arzneimittels, das nach Applikation beim Patienten zu einem Rotigotin-Plasmaspiegel zwischen 0,1 und 15 ng/ml über einen Zeitraum von mindestens 24 Stunden führt.

19. Verwendung nach Anspruch 18, wobei die Depotform zur Behandlung von Morbus Parkinson oder Restless Leg geeignet ist.

20. Verwendung nach einem der vorhergehenden Ansprüche, **dadurch gekennzeichnet, daß** die Depotform zur subkutanen Verabreichung bestimmt ist.

21. Verwendung nach einem der vorhergehenden Ansprüche, **dadurch gekennzeichnet, daß** die Depotform zur intramuskulären Verabreichung bestimmt ist.

22. Verwendung nach einem der vorhergehenden Ansprüche, **dadurch gekennzeichnet, daß** Rotigotin in fester Phase und in einer hydrophoben flüssigen Phase suspendiert vorliegt.

23. Verwendung von Rotigotin in fester Phase zur Herstellung eines hitzesterilisierbaren Arzneimittels, **dadurch gekennzeichnet, dass** beim Erhitzen des Arzneimittels auf 121°C über 20 Minuten bei 0,2 mPa weniger als 1 % des Rotigotins abgebaut wird.

24. Verwendung nach Ansprüchen 18-23, **dadurch gekennzeichnet, dass** Rotigotin kristallin vorliegt.

25. Herstellung einer sterilen pharmazeutischen Zusammensetzung enthaltend Rotigotin, **gekennzeichnet durch** das Autoklavieren einer pharmazeutischen Zusammensetzung, die Rotigotin in fester Phase enthält.

26. Kit zur Behandlung von Morbus Parkinson oder Restless Leg, umfassend
(a) eine pharmazeutische Zusammensetzung nach einem der vorhergehenden Ansprüche
(b) eine Vorrichtung zur Injektion.

## Claims

1. Pharmaceutical composition, **characterized in that** it is an injectable depot form containing N-0923 as the active agent, which after the administration on a patient leads to a rotigotine plasma level of between 0.1 and 15 mg/ml over a period of at least 24 hours.

2. Pharmaceutical composition as in claim 1, **characterized in that** it is a suspension, in which the active ingredient rotigotine is suspended, essentially in its solid phase, in a liquid vehicle.

3. Pharmaceutical composition as in one of the preceding claims, **characterized in that** it is an anhydrous pharmaceutical composition.

4. Pharmaceutical composition as in one of the preceding claims, containing the rotigotine in crystalline form.

5. Pharmaceutical composition as in one of the preceding claims, containing the rotigotine in the form of a crystalline salt.

6. Pharmaceutical composition as in one of the preceding claims **characterized in that** it is an oily suspension.

7. Pharmaceutical composition as in one of the preceding claims, said pharmaceutical composition being an oily suspension whose continuous phase consists essentially of polyol fatty acid esters.

8. Pharmaceutical composition as in claims 6-7, wherein the continuous phase consists essentially of polyol fatty acid esters containing fatty acids with a chain length of 6 to 22 C-atoms.

9. Pharmaceutical composition as in claims 6-8, wherein the continuous phase consists essentially of medium-chain triglycerides.

10. Pharmaceutical composition as in claims 6-9, further **characterized in that** the pharmaceutical composition additionally comprises a wetting agent.

11. Pharmaceutical composition as in claim 10, in which the wetting agent essentially consists of polyol fatty acid esters with a monoester content of over 60%.

12. Pharmaceutical composition as in claims 10-11, in which the wetting agent essentially consists of polyol fatty acid monoesters containing fatty acids with a chain length of 6 to 14 C-atoms.

13. Pharmaceutical composition as in claims 10-12, in which the wetting consists essentially of glycerin monolaurate and/or glycerin monocaprylate.

14. Injectable anhydrous pharmaceutical composition, **characterized in that** it comprises crystalline rotigotine saft in a hydrophobic liquid phase and that upon administration on a patient leads to a continuous plasma level of 0.2 to 10 ng rotigotine/ml over at least 24 hours.

15. Pharmaceutical composition as in one of the preceding claims for subcutaneous or intramuscular administration.

16. Pharmaceutical composition as in one of the preceding claimsfor the treatment of diseases associated with a disorder of the dopamine metabolism.

17. Pharmaceutical composition as in one of the preceding claims, further **characterized in that** the pharmaceutical composition contains at least one additional antiparkinsonian agent.

18. Use of rotigotine or of a rotigotine derivative for producing an injectable depot medication which after the administration on a patient leads to a rotigotine plasma level of between 0.1 and 15 mg/ml over a period of at least 24 hours.

19. Use according to claim 18, wherein the depot medication is suitable for the treatment of Parkinson's Disease or Restless Leg.

20. Use as in one of the preceding claims, **characterized in that** is designed to be applied by subcutaneous administration.

21. Use as in one of the preceding claims, **characterized in that** is designed to be applied by intramuscular administration.

22. Use as in one of the preceding claims, **characterized in that** rotigotine is suspended, essentially in its solid phase, in a liquid hydrophobic phase.

23. Use of rotigotine in its solid phase for producing a heat-sterilizable medicament, **characterized in that**, when the medicament is heated to 121 °C over 20 minutes at 0.2 mPa, less than 1% of the N-0923 decomposes.

24. Use as in claims 18-23, **characterized in that** the rotigotine is in crystalline form.

25. Production of a sterile pharmaceutical composition containing rotigotine, **characterized by** the autoclaving of a pharmaceutical composition containing rotigotine in its solid phase.

26. Kit for treating Parkinson's disease or Restless Leg syndrome, comprising
(a) a pharmaceutical composition as per one of the preceding claims
(b) an injection device.

## Revendications

1. Composition pharmaceutique, ***caractérisée en ce qu'il*** s'agit d'une forme injectable retard contenant le principe actif rotigotine, qui conduit après l'application chez le patient à un taux plasmatique de rotigotine compris entre 0,1 et 15 ng/ml sur une période d'au moins 24 heures.

2. Composition pharmaceutique selon la revendication 1, ***caractérisée en ce qu**'*il s'agit d'une suspension dans laquelle le principe actif rotigotine est présent pour l'essentiel en phase solide, qui est mise en suspension dans un excipient liquide.

3. Composition pharmaceutique selon l'une quelconque des revendications précédentes, ***caractérisée en ce que*** la composition pharmaceutique est anhydre.

4. Composition pharmaceutique selon l'une quelconque des revendications précédentes, ***caractérisée en ce que*** la rotigotine est présente sous forme cristalline.

5. Composition pharmaceutique selon l'une quelconque des revendications précédentes, ***caractérisée en ce que*** la rotigotine est présente sous forme de sel cristallin.

6. Composition pharmaceutique selon l'une quelconque des revendications précédentes, ***caractérisée en ce qu*****'**il s'agit d'une suspension huileuse.

7. Composition pharmaceutique selon l'une quelconque des revendications précédentes, ***caractérisée en ce que*** la composition pharmaceutique est une suspension huileuse dont la phase continue est composée pour l'essentiel de polyol-esters d'acides gras.

8. Composition pharmaceutique selon les revendications 6-7, la phase continue étant composée pour l'essentiel de polyol-esters d'acides gras et les acides gras présentant une longueur de chaîne comprise entre 6 et 22 atomes de C.

9. Composition pharmaceutique selon les revendications 6-8, la phase continue étant composée pour l'essentiel de triglycérides à chaîne moyenne.

10. Composition pharmaceutique selon les revendications 6-9, ***caractérisée en ce que*** la composition pharmaceutique comprend en plus un agent tensio-actif.

11. Composition pharmaceutique selon la revendication 10, l'agent tensio-actif étant composé pour l'essentiel de polyol-esters d'acides gras, dont la proportion en monoesters est supérieure à 60 %.

12. Composition pharmaceutique selon les revendications 10-11, l'agent tensio-actif étant composé pour l'essentiel de polyol-monoesters d'acides gras et les acides gras présentant une longueur de chaîne comprise entre 6 et 14 atomes de C.

13. Composition pharmaceutique selon les revendications 10-12, l'agent tensio-actif étant composé pour l'essentiel de monolawate de glycérine et/ou de monocaprylate de glycérine.

14. Composition pharmaceutique injectable dépourvue d'eau, ***caractérisée en ce qu*****'**elle comprend un sel cristallin de rotigotine qui est mis en suspension dans une phase liquide hydrophobe et qui conduit après administration au patient à un taux plasmatique continu compris entre 0,2 et 10 ng de rotigotine/ml pendant au moins 24 heures.

15. Composition pharmaceutique selon l'une quelconque des revendications précédentes, destinée à une administration sous-cutanée ou intramusculaire.

16. Composition pharmaceutique selon l'une quelconque des revendications précédentes pour le traitement de maladies qui sont liées à des troubles du métabolisme de la dopamine.

17. Composition pharmaceutique selon l'une quelconque des revendications précédentes, ***caractérisée en ce que*** la composition pharmaceutique comprend au moins un autre agent antiparkinsonien.

18. Utilisation de rotigotine ou d'un dérivé de la rotigotine pour la fabrication d'un médicament injectable retard, qui conduit après l'application chez le patient à un taux plasmatique de rotigotine compris entre 0,1 et 15 ng/ml sur une période d'au moins 24 heures.

19. Utilisation selon la revendication 18, la forme retard convenant pour le traitement de la maladie de Parkinson ou le syndrome des jambes sans repos (ou syndrome d'impatience des membres inférieurs).

20. Utilisation selon l'une quelconque des revendications précédentes, ***caractérisée en ce que*** la forme retard est destinée à une administration sous-cutanée.

21. Utilisation selon l'une quelconque des revendications précédentes, ***caractérisée en ce que*** la forme retard est destinée à une administration intramusculaire.

22. Utilisation selon l'une quelconque des revendications précédentes, ***caractérisée en ce que*** la rotigotine est présente en phase solide et est mise en suspension dans une phase liquide hydrophobe.

23. Utilisation de la rotigotine en phase solide pour la fabrication d'un médicament stérilisable à chaud, ***caractérisée en ce que,*** lors du chauffage du médicament à 121 °C pendant 20 min à 0,2 mPa, moins de 1 % de la rotigotine est détruite.

24. Utilisation selon les revendications 18-23, ***caractérisée en ce que*** la rotigotine est présente sous forme cristalline.

25. Fabrication d'une composition pharmaceutique stérile contenant de la rotigotine, ***caractérisée par*** le traitement en autoclave d'une composition pharmaceutique qui contient de la rotigotine en phase solide.

26. Kit de traitement de la maladie de Parkinson ou du syndrome des jambes sans repos, comprenant :
(a) une composition pharmaceutique selon l'une quelconque des revendications précédentes,
(b) un dispositif d'injection.
